(19) Europäisches Patentamt
European Patent Office
Office européen des brevets

(11) Veröffentlichungsnummer: **0 302 486 B1**

(12) **EUROPÄISCHE PATENTSCHRIFT**

(45) Veröffentlichungstag der Patentschrift: **18.03.92**

(51) Int. Cl.$^5$: **C07C 41/28**, C07C 43/15

(21) Anmeldenummer: **88112673.4**

(22) Anmeldetag: **04.08.88**

# (54) Verfahren zur Herstellung von 1.2-Dialkoxyethenen.

(30) Priorität: **06.08.87 DE 3726126**

(43) Veröffentlichungstag der Anmeldung:
**08.02.89 Patentblatt 89/06**

(45) Bekanntmachung des Hinweises auf die Patenterteilung:
**18.03.92 Patentblatt 92/12**

(84) Benannte Vertragsstaaten:
**BE DE FR GB IT NL**

(56) Entgegenhaltungen:
**EP-A- 0 197 283**
**EP-A- 0 217 089**
**EP-A- 0 254 976**
**DE-A- 1 957 680**
**GB-A- 2 091 259**
**US-A- 2 479 068**
**US-A- 3 285 967**

**PATENT ABSTRACTS OF JAPAN, unexamined applications, C Field, Band 7, nr. 28, 4. Februar 1983; THE PATENT OFFICE JAPANESE GOVERNMENT S. 137 C 149**

**DIE ANGEWANDTE MAKROMOLEKULARE CHEMIE, Band 87, 1. Juli 1980, "Polymerisation von 1,2-Dimethoxyethylen" (G. BIER et al); HÜTHIG & WEPF VERLAG, Basel, S. 137-147**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Koeffer, Dieter, Dr.**
**Neuhoefer Strasse 30**
**W-6700 Ludwigshafen(DE)**
Erfinder: **Bertleff, Werner, Dr.**
**Neuzenlache 3**
**W-6806 Viernheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Verfahren zur Herstellung von 1,2-Dialkoxyethenen durch Abspaltung von Alkoholen aus 1,1,2-Trialkoxyalkanen.

Dialkoxyethene finden z.B. als Monomere oder Co-Monomere Verwendung. Die Polymerisation gelingt in Gegenwart von Lewis-Säuren. Zur Darstellung von Dialkoxyethenen sind im wesentlichen zwei Wege bekannt. H. Baganz et al. beschreiben im Chem. Ber., 96, 2657 (1963) die Synthese von 1,2-Dialkoxyethenen aus 1,2-Dialkoxy-1,2-dichlorethan durch Chlor-Eliminierung mit Magnesium. Dabei tritt ein Zwangsanfall von unerwünschtem Magnesiumchlorid auf.

Der zweite Weg zur Darstellung von Dialkoxyethenen ist die Alkohol-Eliminierung aus 1,1,2-Trialkoxyethanen. Vorteilhaft wird die Spaltung des Trialkoxyethans in der Gasphase an Festbettkontakten durchgeführt. So ist aus US-PS 2 479 068 bekannt, daß 1,1,2-Trialkoxyethane bei Temperaturen von 300 bis 450 °C an Bariumhydroxid-belegtem Silicagel zu 1,2-Dialkoxyethenen gespalten werden können. H. Baganz et al. beschreiben die Verwendung von Cerdioxid oder Magnesiumoxid-dotiertem Eisenoxid auf Bimsstein bei Temperaturen zwischen 290 und 360 °C [Chem. Ber., 86, 148 - 154 und 395 - 400 (1953)]. Hierbei werden jedoch nur 27 % Ausbeute erzielt.

Aus US-A-2 479 068 ist die Reaktion von Trialkoxyverbindungen zu ungesättigten Dialkoxyverbindungen bekannt. Als Katalysatoren werden hier $Ba(OH)_2$ auf $SiO_2$ bzw. $Na_2B_4O_7$ auf $SiO_2$ bzw. $SiO_2$ allein verwendet, wobei $Ba(OH)_2SiO_2$ als "outstanding" hervorgehoben wird. Weiterhin werden gefällte B- und Al-Phosphate, $Al_2O_3$, $SiO_2$ und saure "clay like materials", wie man sie in der Petrochemie für Crackreaktionen verwendet, genannt.

Aus EP-A-217 089 ist die katalytische Dealkoxylierung von geminalen Dialkoxyverbindungen an Na-Mordenit und Na-ZSM-5 mit einem $Na_2O/Al_2O_3$-Mol-Verhältnis von 1 : 1,05 (± 0,25) bekannt. Aus den Beispielen sind Molverhältnisse $Na_2O$ zu $Al_2O_3$ im Bereich von 0,75 bis 1,63 bekannt, d.h. es handelt sich um schwach saure oder neutrale bis stark basischen Mordenite. Für ZSM-5 sind Molverhältnisse von 0,95 bis 1,05 bekannt (neutral bis schwach basisch). Beste Ergebnisse hinsichtlich Selektivität werden hier mit basischem Mordenit erhalten.

Aus US-A-3 285 967 ist die Dealkoxylierung von geminalen Dialkoxy-Verbindungen zur Herstellung ungesättigter Vinylester an $M_3PO_4$ (M = Li, K, Na, $\frac{1}{2}$ Mg, $\frac{1}{2}$ Ba, $\frac{1}{2}$ Sr) bzw. auf Silica-träger, insbesondere basisches $Li_3PO_4$, bekannt.

Bei der JP 57-185 232 handelt es sich um dieselbe Reaktion wie in US-A-3 285 967. Hier wird lediglich $Li_3PO_4$ als Katalysator angegeben.

Weiterhin ist bekannt, daß mit Basen vorbehandeltes Aluminiumoxid als Katalysator verwendet werden kann. Die Reaktionstemperatur für die Methanol-Eliminierung aus 1,1,2-Trimethoxyethan zu 1,2-Dimethoxyethen beträgt 300 °C [J. Chem. Eng. Data, 18, 441 (1973)]. Bei einer Ausbeute von 87 % wird ein cis/trans-Verhältnis von 8 eingestellt.

Vergleichbare Ergebnisse ergab ein mit Natriumhydrogensulfat belegter Aluminiumoxid-Kontakt (G. Bier and N. Vollkommer, Angew. Makromol Chem., 87, 137 (1980)). Die erforderliche Reaktionstemperatur für die Herstellung von 1,2-Dimethoxyethen aus 1,1,2-Trimethoxyethan wird mit 350 °C angegeben.

Es bestand die Aufgabe, 1,2-Dialkoxyethene ohne Folgereaktionen an der Doppelbindung oder an der Alkoxygruppe auf einfachem Wege aus leicht zugänglichen Ausgangsstoffen herzustellen, wobei ein möglichst hoher Umsatz und hohe Selektivität des Katalysators bei möglichst langen Standzeiten angestrebt würden.

Es wurde nun gefunden, daß man bei der Herstellung von 1,2-Dialkoxyethenen der Formel (I),

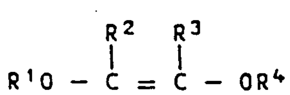

(I)

in der $R^1$ bis $R^4$ untereinander gleich oder verschieden sind und geradkettige oder verzweigte Alkylreste oder Cycloalkylreste oder $R^2$ und $R^3$ auch Wasserstoff oder Arylrest oder Alkenylarylreste bedeuten können, wobei der Aromat durch weitere unter den Reaktionsbedingungen inerte Reste wie Alkyl- oder Alkoxylreste oder Halogen substituiert sein kann, verbesserte Umsätze und Selektivitäten bei langen Standzeiten erzielt, wenn man Alkohole der Formel $R^5OH$ oder $R^4OH$ aus 1,1,2-Trialkoxyethanen der Formel (II),

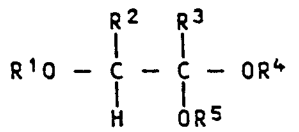

(II)

in der $R^1$ bis $R^4$ obige Bedeutung haben und $R^5$ ein geradkettiger oder verzweigter Alkyl- oder Cycloalkylrest ist, in Gegenwart von Zeolithen und/oder Zr-, Fe- oder Sr-Phosphaten und/oder hydrothermal hergestellten Phosphaten als Katalysatoren abspaltet.

Ein weiterer Vorteil des erfindungsgemäßen Verfahren besteht darin, daß auch die Isomerenverteilung des cis/trans-Produktgemisches über den Katalysator in vorteilhafter Weise steuerbar ist.

Als Reste $R^2$ und $R^3$ kommen unabhängig von $R^1$, $R^4$ und $R^5$ Wasserstoff sowie geradkettige oder verzweigte Alkylreste mit 1 bis 12, insbesondere 1 bis 8, vorzugsweise 1 bis 4 Kohlenstoffatomen in Betracht. Alkylreste sind z.B. Methyl-, Ethyl-, n-Butyl-, i-Butyl-, Pentyl-, Hexyl-, Octyl- oder Decylreste.

Cycloalkylreste sind z. B. Cyclopentyl-, Cyclohexyl-, Cycloheptyl-, Cyclopentenyl- oder Cyclohexenylreste.

Aromatische Reste sind z. B. Phenyl-, Benzyl-, Toluyl- oder Phenylethylreste.

Als Rest $OR^1$ oder $OR^4$ oder $OR^5$ kommen z. B. Methoxy-, Ethoxy-, n/i-Propoxy-, n-/i-/t-Butoxy-, Hexoxyreste in Betracht.

Beispielsweise sind folgende Verbindungen einsetzbar: 1,1,2-Trimethoxyethan, 1,1,2-Triethoxyethan, 1,1-Dimethoxy-2-ethoxyethan, 1,1,2-Tripropoxyethan, 1,1,2-Tributoxyethan und 1,1-Diethoxy-2-propoxyethan.

Als Katalysatoren für das erfindungsgemäße Verfahren werden vorteilhaft acide zeolithische Katalysatoren eingesetzt. Zeolithe sind kristalline Aluminosilikate, die eine hochgeordnete Struktur mit einem starren dreidimensionalen Netzwerk von $SiO_4$ und $AlO_4$-Tetraedern besitzen, die durch gemeinsame Sauerstoffatome verbunden sind. Das Verhältnis der Si- und Al-Atome zu Sauerstoff beträgt 1 : 2 (siehe Ullmanns Encyclopädie d. Techn. Chemie, 4. Auflage, Band 24, Seite 575 (1983)). Die Elektrovalenz der Aluminium enthaltenden Tetraeder ist durch Einschluß von Kationen in den Kristall, z. B. Eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie z. B. B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z. B. in Form eines Kuboktaeders, der aus Vierringen bzw. Sechsringen aufgebaut ist. Je nach Verknüpfung der Kuboktaeder, wodurch unterschiedlich große Hohlräume und Poren entstehen, unterscheidet man Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit-bzw. Chabasit-Type oder Zeolithe vom Faujasit-Typ. z. B. Y-, X- oder L-Zeolithe. In diese Gruppe von Zeolithen gehören auch die sogenannten "ultrastabile" Zeolithe des Faujasittyps, d.h. dealuminierte Zeolithe. Verfahren zur Herstellung solcher Zeolithe sind beschrieben in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch eine hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z. B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminiumsilikatzeolithe haben je nach Wahl der Einsatzstoffmengen ein $SiO_2/Al_2O_3$-Verhältnis von 10 bis 40 000. Derartige Aluminosilikatzeolithe kann man auch in etherischem Medium wie

Diethylenglykoldimethylether, in alkoholischem Medium wie Methanol bzw. 1,4-Butandiol oder in Wasser synthetisieren.

Der Borosilikatzeolith wird z. B. Bei 90 bis 200 ° C unter autogenem Druck synthetisiert, indem man eine Borverbindung z. B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören die isotaktischen Borosilikatezeolithe nach EP 34 727 und EP 46 504. Solche Borosilikatzeolithe können ebenfalls hergestellt werden, wenn man die Reaktion statt in wäßriger Aminlösung in etherischer Lösung, z. B. Diethylenglykoldimethylether oder in alkoholischer Lösung, z. B. 1,6-Hexandiol durchführt.

Eisensilikatzeolithe erhält man z. B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 ° C unter autogenem Druck.

Zu den verwendbaren siliciumreichen Zeolithen ($SiO_2/Al_2O_3 \geqq 10$) gehören auch die sog. ZSM-Typen, Ferrierit, NU-1 und Silicalit®, ein Molekularsieb, ein sog. Silica Polymorph.

Die so hergestellten Alumino-, Boro- und Eisensilikatzeolithe können nach ihrer Isolierung, Trocknung bei 100 bis 160 ° C, vorzugsweise 110 ° C und Calcinierung bei 450 bis 550 ° C, vorzugsweise 500 ° C, mit einem Bindemittel im Verhältnis 90 : 10 bis 40 : 60 Gew.% zu Strängen oder Tabletten verformt werden. Als Bindemittel eignen sich verschiedene Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 ° C/16 h getrocknet und bei 500 ° C/16 h calciniert.

Man erhält auch gut geeignete Katalysatoren, wenn z. B. Der isolierte Alumino- bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z. B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z. B. in der Na-Form, dann kann diese durch Ionenaustausch z. B. mit Ammoniumionen und anschließende Calcinierung oder durch Behandlung mit Säuren vollkommen oder partiell in die gewünschte H-Form überführt werden.

Wenn bei der Anwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 ° C, bevorzugt 500 ° C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Fr, Yb und U eingesetzt.

Zweckmäßigerweise führt man die Dotierung so durch, daß man den verformten Zeolithen in einem Steigrohr vorlegt und bei 20 bis 100 ° C bzw. eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z. B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z. B. darin, daß man $Cu(NO_3)_2 \times 3\ H_2O$ oder $Ni(NO_3)_2 \times 6\ H_2O$ oder $Ce(NO_3)_3 \times 6\ H_2O$ oder $La(NO_3)_2 \times 6\ H_2O$ oder $Cs_2CO_3$ in Wasser löst. Mit dieser Lösung wird der verformte oder unverformte Zeolith eine gewisse Zeit, etwa 30 Minuten, getränkt. Die eventuell überstehende Lösung wird am Rotationsverdampfer von Wasser befreit. Danach wird der getränkte Zeolith bei etwa 150 ° C getrocknet und bei etwa 550 ° C calciniert. Dieser Tränkvorgang kann mehrmals hintereinander

vorgenommen werden, um den gewünschten Metallgehalt einzustellen.

Es ist auch möglich, eine wäßrige $Ni(CO_3)_2$-Lösung oder ammoniakalische $Pd(NO_3)_2$-Lösung herzustellen und darin den reinen pulverförmigen Zeolithen bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z. B. eine wäßrige $Ni(NO_3)_2$-Lösung oder ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten Zeolithen z. B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z. B. 1 bis 3 Stunden bei Temperaturen von 60 bis 80°C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit 0,001 n bis 2 n Flußsäure, vorzugsweise 0,05 n bis 0,5 n Flußsäure behandelt, beispielsweise durch Erhitzen unter Rückfluß über einen Zeitraum von 0,5 bis 5, vorzugsweise 1 bis 3 Stunden. Nach Isolierung, durch Abfiltrieren und Auswaschen, des zeolitischen Materials wird dieses zweckmäßig, bei Temperaturen von 100 bis 160°C, getrocknet und bei Temperaturen von 450°C bis 600°C calciniert. Gemäß einer weiteren bevorzugten Ausführungsform für die Säurebehandlung wird das zeolitische Material nach einer Verformung mit Bindemittel bei erhöhter Temperatur, zweckmäßig bei 50°C bis 90°C, vorzugsweise 60°C bis 80°C, über einen Zeitraum von 0,5 bis 5, mit 12 bis 20 gew.%iger Salzsäure, behandelt. Anschließend wird das zeolithische Material ausgewaschen und zweckmäßig, bei Temperaturen von 100 bis 160°C, getrocknet und bei Temperaturen von 450 bis 600°C calciniert. Einer HF-Behandlung kann sich auch eine HCl-Behandlung anschließen.

Weitere Katalysatoren für das erfindungsgemäße Verfahren sind Phosphate, insbesondere Aluminiumphosphate, Siliciumaluminiumphosphate, Siliciumeisenaluminiumphosphate, Cerphosphat, Zirkonphosphate, Borphosphat, Eisenphosphat oder deren Gemische.

Als Aluminiumphosphat-Katalysatoren werden für das erfindungsgemäße Verfahren insbesondere unter hydrothermalen Bedingungen synthetisierte Aluminiumphosphate eingesetzt.

Die unter hydrothermalen Bedingungen hergestellten Aluminiumphosphate sind z. B. APO-5, APO-9, APO-11, APO-12, APO-14, APO-21, APO-25, APO-31 und APO-33. Synthesen dieser Verbindungen sind in EP 132 708, US 4 310 440 und US 4 473 663 beschrieben.

Beispielsweise das $AlPO_4$-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und danach bei etwa 150°C 20 bis 60 h unter autogenem Druck in einem Autoklaven umsetzt. Das abfiltrierte $AlPO_4$ wird getrocknet bei 100 bis 160°C und calciniert bei 450 bis 550°C.

$AlPO_4$-9 (APO-9) wird ebenfalls aus Orthophosphorsäure und Pseudoboehmit aber in wäßriger DABCO-Lösung (1,4-Diazabicyclo-(2,2,2)octan bei etwa 200°C unter autogenem Druck während 200 bis 400 h synthetisiert.

Die Synthese des $AlPO_4$-21 (APO-21) erfolgt aus Orthophosphorsäure und Pseudoboehmit in wäßriger Pyrrolidon-Lösung bei 150 bis 200°C unter autogenem Druck während 50 bis 200 h.

Die für das erfindungsgemäße Verfahren eingesetzten Siliciumaluminiumphosphate sind z. B. SAPO-5, SAPO-11, SAPO-31 und SAPO-34. Die Synthese dieser Verbindung wird z. B. in EP 103 117, US 4 440 871 beschrieben. SAPO's werden hergestellt durch Kristallisation aus wäßriger Mischung bei 100 bis 250°C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$, suspendiert in wäßriger Tetrapropylammoniumhydroxid-Lösung, mit einer wäßrigen Suspension aus Pseudoboehmit und Orthophosphorsäure und anschließende Umsetzung bei 150 bis 200°C während 20 bis 200 h unter autogenem Druck in einem Rührautoklaven erhalten. Die Trocknung des abfiltrierten Pulvers erfolgt bei 110 bis 160°C

und die Calcination bei 450 bis 550 °C.

Als Phosphatkatalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g $Al(NO_3)_3$ x $H_2O$ in 700 ml Wasser 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60 °C/16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcinierung in Inertgas-, Luft- oder Dampfatmosphäre bei 250 bis 650 °C vorzugsweise 300 bis 500 °C herstellen.

Auf diese Phosphate können auch Imprägnierung (Tränken und Aufsprühen) oder in manchen Fällen auch durch Ionenaustausch Modifizierungskomponenten, wie voran bei den Zeolithen beschrieben, aufgebracht werden. Auch kann bei den Zeolithkatalysatoren eine Modifizierung mit Säuren erfolgen.

Geeignete saure, unbehandelte Katalysatoren sind z. B. Auch die sauer wirkenden Oxide der Elemente Ti, Zr, Si, Al, V, W, Mo, Nb und Cr. Es sind dies Titandioxid, Zirkondioxid, Vanadiumoxide, Nioboxide, Chromoxide, Molybdänoxide, Wolframoxide oder Gemische dieser Oxide. So kann man nach dem erfindungsgemäßen Verfahren mit diesen Katalysatoren in guten Ausbeuten durch Abspaltung von Methanol aus 1,1,2-Trimethoxyethan das 1, 2-Dimethoxyethen herstellen.

Auch mit Phosphorsäure oder Borsäure getränkte Katalysatoren können eingesetzt werden. Phosphorsäure oder Borsäure wird z. B. auf $SiO_2$-, $Al_2O_3$- oder Bimsträger, z. B. durch Auftränken oder Versprühen aufgebracht. Ein phosphorsäurehaltiger Katalysator kann beispielsweise durch Auftränken von $H_3PO_4$ auf $SiO_2$ und anschließende Trocknung bzw. Calcination erhalten werden. Phosphorsäure kann aber auch mit Kieselgel zusammen in einem Sprühturm versprüht werden; danach schließt sich eine Trocknung und meist eine Calcination an. Phosphorsäure kann auch in einer Imprägniermühle auf das Trägermaterial aufgesprüht werden.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die erfindungsgemäße Umwandlung wird in der Regel bevorzugt in der Gasphase bei 100 bis 450 °C insbesondere 150 bis 350 °C, vorzugsweise bei 250 - 300 °C, und einer Belastung WHSV = 0,1 bis 20 $h^{-1}$, bevorzugt 0,5 bis 5 $h^{-1}$ (g Ausgangsgemisch/g Katalysator und Stunde) in einem Festbett oder auch im Wirbelbett ausgeführt.

Es ist auch möglich, die Reaktion in der Flüssigphase (Suspension-, Riesel- oder Sumpffahrweise) bei Temperaturen zwischen 50 und 200 °C durchzuführen.

Das Verfahren wird in der Regel bei Normaldruck oder je nach Flüchtigkeit der Ausgangsverbindung bei vermindertem oder erhöhtem Druck durchgeführt, wobei die Durchführung vorzugsweise kontinuierlich, aber auch diskontinuierlich erfolgen kann.

Schwerflüchtige oder feste Ausgangsstoffe werden in gelöster Form z. B. in THF-, Toluol- oder Petrolether-Lösung zum Einsatz gebracht. Generell ist eine Verdünnung des Eduktes mit derartigen Lösungsmitteln oder mit Inertgasen wie $N_2$, Ar, $H_2O$-Dampf möglich.

Nach der Umsetzung werden die entstandenen Produkte durch übliche Verfahren, z. B. durch Destillation, aus dem Reaktionsgemisch isoliert; nichtumgesetzte Ausgangsstoffe werden gegebenenfalls in die Umsetzung zurückgeführt.

Vorzugsweise werden die gasförmigen Reaktionsprodukte sofort in eine Trennung eingebracht und z. B. in einer Fraktionierkolonne in ihre Einzelkomponenten zerlegt, um eine Rückreaktion zu unterbinden und um einen hohen Umsatz zu erzielen. Durch einen möglichst vollständigen Umsatz wird die Aufarbeitung des Produktgemisches erleichtert, insbesondere bezüglich der Trennung von Trimethoxyethan und Dimethoxyethylen.

Beispiele 1 - 23

Die Reaktionen in der Gasphase werden unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8 000 g einer wäßrigen 1,6-Hexandiamin-Lösung (Mischung 50 : 50 Gew.%) bei 170° C unter autogenem Druck in einem Rührautoklaven hergestellt. Nach Abfiltrieren und Auswaschen wird das kristalline Reaktionsprodukt bei 100° C/24 h getrocknet und bei 500° C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit einem Verformungshilfsmittel 2 mm-Stränge hergestellt, die bei 110° C/16 h getrocknet und bei 500° C/24 h calciniert werden.

Katalysator B

Ein Aluminosilikatzeolith vom Pentasil-Typ wird unter hydrothermalen Bedingungen bei autogenem Druck und 150° C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50 : 50 Gew.%) in einem Rührautoklaven hergestellt. Nach Abfiltrieren und Auswaschen wird das kristalline Reaktionsprodukt bei 110° C/24 h getrocknet und bei 500° C/24 h calciniert. Dieser Aluminosilikatzeolith enthält 91,6 Gew.% $SiO_2$ und 4,6 Gew.% $Al_2O_3$. Der Katalysator wird mit einem Verformungshilfsmittel zu 2 mm-Strängen verformt, bei 110° C/16 h getrocknet und bei 500° C/24 h calciniert.

Katalysator C

Katalysator C wird erhalten, indem man die Stränge des Katalysators A mit einer wäßrigen $Cs_2CO_3$-Lösung imprägniert, danach bei 130° C/2 h trocknet und bei 540° C/2 h calciniert. Der Cs-Gehalt beträgt 0,6 Gew.%.

Katalysator D

200 g des bei Katalysator A beschriebenen Borosilikatzeolith werden mit 1 l einer wäßrigen Lösung aus 16,7 g $FeCl_3$ x 6 $H_2O$ und 50 g $NH_4Cl$ 24 h bei Raumtemperatur ionenausgetauscht, danach gründlich mit $H_2O$ Cl-frei gewaschen, bei 150° C/1 h getrocknet und bei 500° C/2 h calciniert. Dieses Pulver wird mit hochdispersem $SiO_2$ im Gewichtsverhältnis 70 : 30 verformt. Nach Trocknung werden die Stränge bei 500° C/16 h calciniert.

Katalysator E

Katalysator E wird erhalten, indem man die Stränge des Katalysators A mit einer wäßrigen Lösung aus Cer- und Palladiumnitrat imprägniert, danach bei 130° C/2 h trocknet und bei 540° C/2 h calciniert. Der Ce-Gehalt beträgt 2,3 Gew.%, der Pd-Gehalt 0,5 Gew.%.

Katalysator F

Katalysator F wird wie Katalysator C hergestellt, wobei jedoch $Cs_2CO_3$ durch $Fe(NO_3)_3$ ersetzt wird. Der Fe-Gehalt beträgt 2,9 Gew.%.

Katalysator G

Der Eisensilikatzeolith des Pentasil-Typs wird unter hydrothermalen Bedingungen bei autogenem Druck und 165° C aus 273 g Wasserglas, gelöst in 253 g einer wäßrigen 1,6-Hexandiamin-Lösung (Mischung 50 : 50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110° C/24 h getrocknet und bei 500° C/24 h calciniert. Man erhält einen Eisensilikatzeolithen mit einem $SiO_2/Fe_2O_3$-Verhältnis von 17,7 und einen $Na_2O$-Gehalt von 1,2 Gew.%. Der Katalysator wird mit hochdispersem $SiO_2$ im Gewichtsverhältnis 70 : 30 zu 2,5 mm-Strängen gepreßt, bei 110° C/16 h getrocknet und bei 500°/24 h calciniert. Diese Stränge werden mit einer 20 %igen $NH_4Cl$-Lösung bei 80° C ionenausgetauscht und danach chloridfrei gewaschen, bei 110° C getrocknet und bei 500°/5 h calciniert. Der Ionenaustausch wird so lange vorgenommen, bis der Na-Gehalt 0,002 Gew.% beträgt.

Katalysator H

Katalysator H wird wie Katalysator C hergestellt, wobei jedoch $Cs_2CO_3$ durch $Ce(NO_3)_2$ ersetzt wird. Der Ce-Gehalt beträgt 1,8 Gew.%.

Katalysator I

Wie Katalysator H, nur 1,2 Gew.% Ce.

Katalysator J

Katalysator J wird wie Katalysator C hergestellt, wobei jedoch $Cs_2CO_3$ durch $Cr(NO_3)_3$ ersetzt wird. Der Cr-Gehalt beträgt 1,9 Gew.%.

Katalysator K

$AlPO_4$-12 (APO-12) wird synthetisiert, indem man 200 g 98 %ige Phosphorsäure und 136 g Boehmit in 400 Wasser löst bzw. suspendiert, hierzu eine wäßrige Lösung aus 60 g Ethylendiamin und 320 g $H_2O$ zugibt und diese Mischung in einem Rührautoklaven bei 200 ° C während 24 Std. unter autogenem Druck umsetzt. Nach Abfiltrieren wird das kristalline Material bei 120 ° C getrocknet und bei 500 ° C/16 h calciniert. Das so synthetisierte $AlPO_4$-12 enthält 55,5 Gew.% $P_2O_5$, 39,7 Gew.% $Al_2O_3$. Dieses Material wird mit Verstrangungshilfsmitteln zu 3 mm-Strängen verformt, abermals bei 120 ° C getrocknet und bei 500 ° C/6 h calciniert.

Katalysator L

Die Synthese von $AlPO_4$-5 (APO-5) erfolgt durch Zusammenrühren von 200 g 95 %iger Phosphorsäure gelöst in 325 g $H_2O$, 136 g Boehmit und 678 g Tetrapropylammoniumhydroxid (30 %ig) und anschließende Reaktion bei 150 ° C unter autogenem Druck während 43 Stunden. Das bei 120 ° C getrocknete und bei 500 ° C/16 h calcinierte Produkt enthält 46,5 Gew.% $P_2O_5$ und 45,5 Gew.% $Al_2O_3$. Dieses $AlPO_4$-5 wird mit Boehmit im Gewichtsverhältnis 60 : 40 zu 2 mm-Strängen verformt, bei 110 ° C getrocknet und bei 500 ° C/16 h calciniert

Katalysator M

Siliciumaluminiumphosphat-5 (SAPO-5) wird hergestellt aus einer Mischung aus 200 g 98 %ige Phosphorsäure, 136 g Boehmit, 60 g Kieselsol (30 %ig) 287 g Tripropylamin und 587 g $H_2O$. Diese Mischung wird bei 150 ° C während 168 Std. unter autogenem Druck umgesetzt. Nach Filtration wird das kristalline Produkt bei 120 ° C getrocknet und bei 500 ° C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3 mm-Strängen verformt, bei 120 ° C getrocknet und bei 500 ° C calciniert.

Katalysator N

Kommerziell erhältliches Zirkonphosphat $Zr_3(PO_4)_4$ in Reinsubstanz verformt.

Vergleichskatalysator O

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3 mm-Strängen verformt. Diese Stränge werden bei 100 ° C getrocknet und bei 350 ° C calciniert. Katalysator O enthält 8,77 Gew.% B und 28,3 Gew.% P.

Vergleichskatalysator P

Katalysator P ist ein gefälltes Aluminiumphosphat, das durch Fällung aus $Al(NO_3)_3$-$H_3PO_4$-Lösung mit $NH_3$ bei pH = 6 - 7 erhalten wird. Nach Abfiltrieren des Niederschlags wird bei 110 ° C getrocknet und bei 500 ° C calciniert. Katalysator P enthält 28,5 Gew.% Al und 13,2 Gew.% P.

Katalysator Q

Kommerziell erhältlicher NaY-Zeolith wird mit Verformungshilfsmittel zu 2 mm verstrangt, bei 110°C getrocknet und bei 500°C/16 h calciniert und mit 20 %iger Ammoniumchloridlösung einem Ionenaustausch unterworfen. Der Restnatriumgehalt des Katalysators Q beträgt 0,85 Gew.% (500°C calciniert).

Katalysator R

Katalysator R ist kommerziell erhältlicher Mordenit (Zeolon 900 H®) in der H-Form.

Vergleichskatalysator S

$TiO_2$ P 25® wird zu 2 mm-Stränge verformt, bei 110°C getrocknet und bei 500°C/16 h calciniert.

Vergleichskatalysator T

$SiO_2$ im Handel erhältlich D 11-10®.

Vergleichskatalysator U

D 10-10® $Al_2O_3$ wird mit $H_3BO_3$ imprägniert, getrocknet bei 110°C und bei 500°C/5 h calciniert. Der Katalysator U setzt sich zusammen aus 85 % $Al_2O_3$ und 15 % $B_2O_3$.

Die mit diesen Katalysatoren erzielten Versuchsergebnisse und Versuchsbedingungen sind in Tabelle 1 zusammengefaßt.

Aus Tabelle 1 erkennt man, daß das cis/trans-Verhältnis durch die Wahl des Katalysators beeinflußt werden kann; mit den Phosphat-Katalysatoren erhält man im allgemeinen mehr trans-Verbindung. Die Selektivität liegt bei den Phosphaten mit Zeolithstruktur höher als bei den Phosphaten ohne Zeolithstruktur.

Tabelle 1: Trimethoxyethan(II) ⟶ Dimethoxyethylen(I) + Methanol

| Beispiel | 1[1] | 2[1] | 3[2] | 4[1] | 5[2] | 6[1] | 7[1] | 8[1] | 9[1] | 10[1] | 11[1] |
|---|---|---|---|---|---|---|---|---|---|---|---|
| Katalysator | A | A | A | B | C | D | E | F | G | H | I |
| Temperatur | 250 | 300 | 250 | 300 | 300 | 300 | 300 | 300 | 300 | 300 | 300 |
| WHSV $h^{-1}$ | 3,5 | 3,5 | 2,5 | 2 | 1 | 2 | 1,5 | 2 | 3 | 2 | 3 |
| Umsatz (II) % | 92,8 | 98,0 | 96,9 | 98,4 | 97,6 | 91,0 | 92,1 | 99,0 | 98,4 | 97,4 | 99,5 |
| Selektivität (I) % trans | 32,6 | 33,1 | 32,8 | 28,8 | 33,4 | 32,1 | 23,7 | 32,2 | 31,4 | 33,3 | 31,4 |
| Selektivität (I) % cis | 56,6 | 51,2 | 55,3 | 58,7 | 51,5 | 52,1 | 44,9 | 50,5 | 49,8 | 53,4 | 57,9 |
| Selektivität Σ | 89,2 | 84,3 | 88,1 | 87,5 | 84,9 | 84,2 | 68,6 | 82,7 | 81,3 | 86,7 | 89,3 |

1) THF-Lösung 50 : 50 Gew.%
2) Einsatz als Reinsubstanz

Die Beispiele 19, 20, 21, 22 und 23 sind Vergleichsbeispiele.

Tabelle 1: Trimethoxyethan(II) ⟶ Dimethoxyethylen(I) + Methanol

| Beispiel | 12[1] | 13[1] | 14[1] | 15[1] | 16[1] | 17[1] | 18[1] | 19[1] | 20[1] | 21[1] | 22[1] | 23[1] |
|---|---|---|---|---|---|---|---|---|---|---|---|---|
| Katalysator | J | Q | R | K | L | M | N | O | P | S | T | U |
| Temperatur | 300 | 300 | 300 | 300 | 300 | 300 | 300 | 300 | 300 | 300 | 300 | 300 |
| WHSV $h^{-1}$ | 1 | 1 | 2 | 3 | 2 | 2 | 2 | 2,5 | 1,5 | 1,5 | 3,5 | 2,5 |
| Umsatz (II) % | 98,6 | 89,6 | 55,5 | 91,6 | 98,6 | 65,3 | 81,2 | 85,5 | 98,5 | 84,9 | 98,9 | 99 |
| Selektivität (I) % trans | 28,9 | 23,9 | 29,1 | 23,4 | 29,7 | 25,7 | 22,6 | 19,1 | 32,5 | 15,3 | 27,1 | 27,3 |
| Selektivität (I) % cis | 52,7 | 63,5 | 65,2 | 72,9 | 65,4 | 67,7 | 67,3 | 67,0 | 52,3 | 65,2 | 52,7 | 49,1 |
| Selektivität Σ | 81,6 | 87,4 | 94,3 | 96,3 | 95,1 | 93,4 | 89,9 | 86,1 | 84,8 | 80,5 | 79,8 | 76,4 |

1) THF-Lösung 50 : 50 Gew.%
2) Einsatz als Reinsubstanz

## Patentansprüche

1. Verfahren zur Herstellung von Dialkoxyethenen der Formel (I),

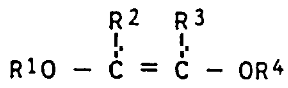

(I)

in der $R^1$ bis $R^4$ untereinander gleich oder verschieden sind und geradkettige oder verzweigte Alkylreste oder Cycloalkylreste oder $R^2$ und $R^3$ auch Wasserstoff oder Arylreste oder Alkenylarylreste bedeuten können, wobei der Aromat durch weitere Reste wie Alkyl- oder Alkoxylreste oder Halogene substituiert sein kann, dadurch gekennzeichnet, daß man Alkohole der Formel $R^4OH$ oder $R^5OH$ aus Trialkoxyethanen der Formel (II),

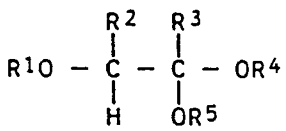

(II)

in der $R^1$ bis $R^4$ obige Bedeutung haben und $R^5$ ein geradkettiger oder verzweigter Alkyl- oder Cycloalkylrest ist, in Gegenwart von Zeolithen und/oder Zr-, Fe- oder Sr-Phosphaten und/oder hydrothermal hergestellten Phosphaten als Katalysatoren abspaltet.

2. Verfahren zur Herstellung von 1,2-Dimethoxyethen, dadurch gekennzeichnet, daß man Methanol aus 1,1,2-Trimethoxyethan in Gegenwart von Zeolithen und/oder Zr-, Fe- oder Sr-Phosphaten und/oder hydrothermal hergestellten Phosphaten als Katalysatoren abspaltet.

3. Verfahren nach Anspruch 1 und 2, dadurch gekennzeichnet, daß man als Katalysatoren Zeolithe des Pentasiltyps verwendet.

4. Verfahren nach Anspruch 1 bis 3, dadurch gekennzeichnet, daß man als Katalysatoren Bor-, Eisen- oder Aluminiumsilikatzeolithe verwendet.

5. Verfahren nach den Ansprüchen 1 bis 3, dadurch gekennzeichnet, daß man als Katalysatoren Aluminiumsilikatzeolithe des Y-Typs verwendet.

6. Verfahren nach Anspruch 1 bis 5, dadurch gekennzeichnet, daß man mit Alkalimetallen, Übergangsmetallen, Seltenen Erdmetallen dotierte Zeolithe als Katalysatoren verwendet.

7. Verfahren nach Anspruch 1 und 2, dadurch gekennzeichnet, daß man als Katalysatoren hydrothermal hergestellte Phosphate, der Elemente B, Al, Zr, Fe, Sr oder deren Gemische verwendet.

8. Verfahren nach Anspruch 1 und 2, dadurch gekennzeichnet, daß man als Katalysatoren hydrothermal hergestellte Aluminiumphosphate oder Siliciumaluminiumphosphate oder Siliciumeisenaluminiumphosphate oder Boraluminiumphosphat verwendet.

9. Verfahren nach Anspruch 1 bis 8, dadurch gekennzeichnet, daß man die Reaktion in der Gasphase durchführt.

## Claims

1. A process for the preparation of a dialkoxyethene of the formula (I)

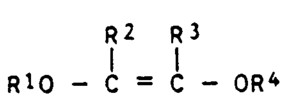

(I)

where $R^1$ to $R^4$ are identical or different and are each straight-chain or branched alkyl or cycloalkyl or $R^2$ and $R^3$ may furthermore be hydrogen, aryl or alkenylaryl, and the aromatic may be substituted by further radicals such as alkyl, alkoxy or halogens, wherein an alcohol of the formula $R^4OH$ or $R^5OH$ is eliminated from a trialkoxyethane of the formula (II)

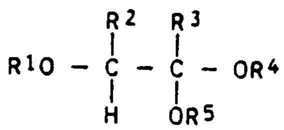

(II)

where $R^1$ to $R^4$ have the above meanings and $R^5$ is straight-chain or branched alkyl or cycloalkyl, in the presence of a zeolite and/or a Zr, Fe or Sr phosphate and/or a hydrothermally prepared phosphate as a catalyst.

**2.** A process for the preparation of a 1,2-dimethoxyethene, wherein methanol is eliminated from 1,1,2-trimethoxyethane in the presence of a zeolite and/or a Zr, Fe or Sr phosphate and/or a hydrothermally prepared phosphate as a catalyst.

**3.** A process as claimed in claim 1 or 2, wherein the catalyst used is a zeolite of the pentasil type.

**4.** A process as claimed in any of claims 1 to 3, wherein the catalyst used is a borosilicate, iron silicate or aluminosilicate zeolite.

**5.** A process as claimed in any of claims 1 to 3, wherein the catalyst used is an aluminosilicate zeolite of the Y type.

**6.** A process as claimed in any of claims 1 to 5, wherein a zeolite doped with an alkali metal, a transition metal or a rare earth metal is used as the catalyst.

**7.** A process as claimed in claim 1 or 2, wherein the catalyst used is a hydrothermally prepared phosphate, of the elements B, Al, Zr, Fe or Sr or a mixture of these.

**8.** A process as claimed in claim 1 or 2, wherein the catalyst used is a hydrothermally prepared aluminum phosphate or silicon aluminum phosphate or silicon iron aluminum phosphate or boron aluminum phosphate.

**9.** A process as claimed in claim 1 to 8, wherein the reaction is carried out in the gas phase.

## Revendications

**1.** Procédé de préparation de dialcoxyéthylènes de formule (I),

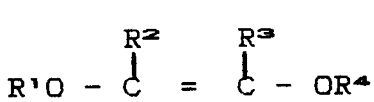

(I)

dans laquelle les substituants $R^1$ à $R^4$ sont identiques ou différents les uns des autres et représentent des restes alkyle à chaîne linéaire au ramifiée ou des restes cycloalkyle, ou bien $R^2$ et $R^3$ peuvent aussi représenter des atomes d'hydrogène ou des restes aryle ou alcénylaryle, le groupement aromatique pouvant être substitué par d'autres restes comme des restes alkyle ou alcoxy ou des atomes d'halogène, caractérisé en ce qu'on sépare des alcools de formule $R^4OH$ ou $R^5OH$ à partir de trialcoxyéthanes de formule (II),

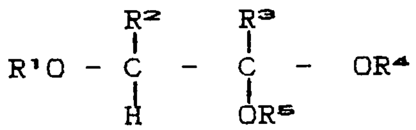 (II)

dans laquelle les substituants $R^1$ à $R^4$ ont les significations données ci-dessus et $R^5$ est un reste alkyle linéaire ou ramifié ou cycloalkyle,
en présence de zéolithes et/au de phosphates de Zr, Fe ou Sr et/ou de phosphates préparés par voie hydrothermale en tant que catalyseurs.

2. Procédé de préparation du 1,2-diméthoxyéthylène, caractérisé en ce qu'on coupe du méthanol à partir du 1,1,2-triméthoxyéthane en présence de zéolithes et/ou de phosphates de Zr, Fe ou Sr et/ou de phosphates préparés par voie hydrothermale.

3. Procédé selon la revendication 1 ou 2, caractérisé en ce qu'on utilise comme catalyseurs des zéolithes du type pentasil.

4. Procédé selon les revendications 1 à 3, caractérisé en ce qu'on utilise comme catalyseurs des zéolithes de borosilicate, de ferrosilicate ou d'aluminosilicate.

5. Procédé selon les revendications 1 à 3, caractérisé en ce qu'on utilise comme catalyseurs des zéolithes d'aluminosilicate du type Y.

6. Procédé selon les revendications 1 à 5, caractérisé en ce qu'on utilise comme catalyseurs des zéolithes dopées avec des métaux alcalins, des métaux de transition et/ou des métaux des terres rares.

7. Procédé selon la revendication 1 ou 2, caractérisé en ce qu'on utilise comme catalyseurs des phosphates préparés par voie hydrothermale des éléments B, Al, Zr, Fe, Sr ou de leurs mélanges.

8. Procédé selon la revendication 1 ou 2 caractérisé en ce qu'on utilise comme catalyseurs des phosphates d'aluminium, des phosphates de silicium et d'aluminium, des phosphates de silicium, de fer et d'aluminium, ou des phosphates de bore et d'aluminium, tous préparés par voie hydrothermale.

9. Procédé selon les revendications 1 à 8, caractérisé en ce qu'on effectue la réaction en phase gazeuse.